# EUROPÄISCHE PATENTSCHRIFT

(11) **EP 2 425 862 B1**
(45) Veröffentlichungstag und Bekanntmachung des Hinweises auf die Patenterteilung: **29.08.2012**
(21) Anmeldenummer: 10401159.8
(22) Anmeldetag: 07.09.2010
(51) Int. Cl.: A61L 2/07, A61L 2/24, A61G 9/02

(54) **Verfahren zur Reinigung und thermischen Desinfektion von Spülgut**
Method of cleaning and thermal disinfection of goods
Procédé de nettoyage et de désinfection thermique de vaisselle

(43) Veröffentlichungstag der Anmeldung: 07.03.2012
(73) Patentinhaber: Miele & Cie. KG, 33332 Gütersloh (DE)
(72) Erfinder: Sedlag, Michael, 48167 Münster (DE); Horstmann, Peter, 33332 Gütersloh (DE); Spaller, Kathleen, 33330 Gütersloh (DE)

(56) Entgegenhaltungen:
- EP-A1- 0 093 846
- WO-A1-2004/016588
- DE-U- 8 122 304
- FR-A1- 2 725 135
- US-A- 4 527 843

## Beschreibung

Die Erfindung betrifft ein Verfahren zur Reinigung und thermischen Desinfektion von Spülgut, wie Steckbecken, Urinflaschen und dergleichen, bei dem das in einer mittels einer Tür verschließbaren Spülgutkammer befindliche Spülgut in einem ersten Verfahrensschritt zwecks Reinigung mit einer Reinigungsflüssigkeit und in einem zweiten Verfahrensschritt zwecks thermischer Desinfektion mit Wasserdampf beaufschlagt wird, wobei nach Abschluss des Desinfektionsschrittes eine Rückkühlung des Spülguts erfolgt, zu welchem Zweck die die Spülgutkammer verschließende Tür um ein vorgebbares Spaltmaß automatisch geöffnet wird.

Verfahren zur Reinigung und thermischen Desinfektion von Spülgut sind aus dem Stand der Technik an sich bekannt, auch in der Anwendung zur Reinigung und thermischen Desinfektion von Steckbecken, Urinflaschen und dergleichen.

Gemäß der vorbekannten Verfahren wird das Spülgut in einem ersten Verfahrensschritt zunächst mit Wasser beaufschlagt. Dieser Verfahrensschritt stellt den eigentlichen Reinigungsvorgang dar. Nach Abschluss dieses Verfahrensschrittes erfolgt in einem zweiten Verfahrensschritt eine Desinfektion des gereinigten Spülgutes, und zwar eine thermische Desinfektion mittels Wasserdampf.

Infolge des Verfahrensschrittes der thermischen Desinfektion heizt sich das Spülgut auf. Vor einer weiteren Verwendung des Spülguts bedarf es deshalb der Abkühlung.

Aus dem Stand der Technik ist es bekannt, das Spülgut zwecks Abkühlung mit Wasser zu besprühen. Diese Vorgehensweise hat sich allerdings aus mehreren Gründen als nachteilig erwiesen. Es stellt sich zum einen ein erhöhter Wasserverbrauch ein. Zum anderen kann das auf das Spülgut aufgebrachte Wasser zu optisch unansehnlichen Rückständen auf dem Spülgut führen, was beim Verwender zu dem Eindruck einer nur unzureichenden Reinigung und/oder Desinfektion des Spülgutes führen kann. Besonders schwerwiegend wirkt indes der Nachteil, dass eine Beaufschlagung des Spülguts mit Wasser zwecks Rückkühlung nach erfolgtem Desinfektionsschritt durchgeführt wird. Infolge der Rückkühlung kann es so zu einer Verkeimung des zuvor thermisch desinfizierten Spülgutes kommen. Diese Verkeimungsgefahr besteht insbesondere dann, wenn zum Ausbringen des Kühlwassers die schon während des Reinigungsschrittes verwendeten Sprühdüsen genutzt werden.

Aus dem Stand der Technik ist gemäß der EP 1 767 226 A1 ferner ein Verfahren zur Reinigung und thermischen Desinfektion von Spülgut bekanntgeworden, bei dem nach erfolgter thermischer Desinfektion eine Rückkühlung des Spülguts mittels in die Spülgutkammer zwangsweise eingebrachter Zuluft erfolgt. Dabei erfolgt das zwangsweise Einbringen von Zuluft in die Spülgutkammer bei geschlossener Tür. Zum Ableiten der Abluft aus der Spülgutkammer ist eine Abluftleitung vorgesehen, die unter Zwischenschaltung eines Abluftventils an den Siphonbogen der Abwasserleitung der Spülgutkammer angeschlossen ist.

Auch die aus der EP 1 767 226 A1 bekannte Verfahrensdurchführung ist nicht frei von Nachteilen. Zum einen macht die aus der EP 1 767 226 A1 bekannte Verfahrensdurchführung einen erhöhten Energieverbrauch erforderlich. Der erhöhte Energieverbrauch ergibt sich dadurch, dass die zur Rückkühlung vorgesehene Zuluft in die geschlossene und mit einer Dampfatmosphäre gefüllte Spülgutkammer entgegen des dort herrschenden Drucks einzubringen ist, was im Übrigen auch ein entsprechend leistungsstarkes Lüftungsaggregat erforderlich macht. Darüber hinaus wird es als nachteilig angesehen, dass die Ableitung der Abluft in den Siphonbogen der Abwasserleitung zu Geruchsbelästigungen führen kann. Es ist zwar gemäß der EP 1 767 226 A1 vorgesehen, die Abluftleitung unter Zwischenordnung eines Abluftventils am Siphonbogen der Abwasserleitung anzuschließen, doch die Abführung der Abluft kann dazu führen, dass es zu einer Aufwirbelung des im Siphonbogen befindlichen Restwassers kommt, was schlussendlich zu einem Geruchseintrag in die Spülgutkammer führen kann. Darüber hinaus stellt das in die Abluftleitung integrierte Abluftventil eine potentielle Stör- und Ausfallquelle dar.

Aus der US 4 527 843 A, der FR 2 725 135 A1, der EP 0 093 846 A1 und der DE 81 22 304 U ist es bekannt, bei den eingangs erwähnten Reinigungsgeräten die Tür am Ende des Reinigungsprozesses motorisch vollständig zu öffnen.

Es ist ausgehend vom Vorbeschriebenen die Aufgabe der Erfindung, ein Verfahren der gattungsgemäßen Art dahingehend zu verbessern, dass bei gleichzeitiger Sicherstellung einer ordnungsgemäßen Desinfektion eine vereinfachte Prozessführung möglich ist.

Mit dem erfindungsgemäßen Verfahren ist vorgesehen, dass nach Abschluss des thermischen Desinfektionsprozesses mittels entspanntem Wasserdampf die Tür der Spülgutkammer automatisch geöffnet und in eine definierte Position verbracht wird, in der sie für eine vorgebbare Zeitspanne verbleibt. Durch die definierte Offenstellung der Tür kann die in der Spülgutkammer befindliche Dampfatmosphäre aus der Spülgutkammer in die Atmosphäre entweichen. Nach Ablauf der vorgebbaren Zeitspanne wird die Tür freigegeben. Es kann dann eine manuelle oder automatische Öffnung und Entladung der Spülgutkammer stattfinden.

Gemäß einem weiteren Merkmal der Erfindung kann nach einem automatischen Öffnen der Tür Zuluft in die Spülgutkammer eingebracht werden. Diese in die Spülgutkammer eingebrachte Zuluft kann die bereits eingesetzte Selbsttrocknung des Spülgutes unterstützen und das Spülgut für eine gefahrlose Entnahme zusätzlich abkühlen. Darüber hinaus wird durch den Eintrag von Zuluft der Austrag der in der Spülgutkammer nach Abschluss der thermischen Desinfektion befindlichen Dampfatmosphäre beschleunigt. Eine insgesamt schnellere Abtrocknung des Spülgutes kann so erreicht werden.

Gemäß einem weiteren Merkmal der Erfindung erfolgt die Dampferzeugung zur thermischen Desinfektion unter Verwendung eines aromatisierten Wasserzusatzes. Diese Ausgestaltung hat insbesondere den Vorteil, dass die nach Abschluss der thermischen Desinfektion innerhalb der Spülgutkammer befindliche Dampfatmosphäre aromatisiert ist. Diese aromatisierte Dampfatmosphäre wird im nachfolgenden Trocknungsschritt aus der Spülgutkammer ausgebracht und in die Atmosphäre abgegeben. Dies führt zu einer Geruchsaufwertung der Umgebungsatmosphäre, was zu einer Aromatisierung des Raumes genutzt werden kann, in dem sich die Vorrichtung zur Verfahrensdurchführung befindet, d. h., in dem das erfindungsgemäße Verfahren durchgeführt wird.

Mit der erfindungsgemäßen Verfahrensdurchführung gehen insbesondere folgende Vorteile einher:
- keine Rückverkeimung des desinfizierten Spülguts
- verbesserte Trocknung des desinfizierten Spülguts
- Wassereinsparung
- Energieeinsparung aufgrund der Selbsttrocknung des aufgeheizten Spülguts und geringerer Zuluftgebläseleistung
- Verbesserung der Geruchsqualität in der Umgebung des der zur Verfahrensdurchführung genutzten Vorrichtung.

Um eine Verkalkung der Heizung im Dampferzeuger zu verhindern, ist der zu verdampfenden Wassermenge regelmäßig ein Entkalkungsmittel zuzusetzen. Dabei kommt bevorzugterweise ein aromatisiertes Entkalkungsmittel zum Einsatz.

Durch Zudosierung eines aromatisierten Entkalkungsmittels entsteht bei der Dampferzeugung zur thermischen Desinfektion ein aromatisierter Dampf. Dieser entweicht während des Trocknungsprozesses über den Öffnungsspalt bei teilweise geöffneter Tür in die Umgebungsatmosphäre. Je nach eingesetzten Aromen können sehr ansprechende Geruchsqualitäten erzeugt werden, die sich im Aufstellungsraum der erfindungsgemäßen Vorrichtung verteilen.

Weitere Vorteile und Merkmale der Erfindung ergeben sich aus der nachfolgenden Beschreibung anhand der Figuren. Dabei zeigen:
- Fig. 1: in schematisch perspektivischer Ansicht eine Vorrichtung nach der Erfindung;
- Fig. 2: in schematisch perspektivischer Ansicht eine Vorrichtung nach der Erfindung mit unter anderem geöffneter Tür;
- Fig. 3: die erfindungsgemäße Vorrichtung in teilgeschnittener Seitenansicht und
- Fig. 4: die erfindungsgemäße Vorrichtung in Seitenansicht mit geöffneter Tür.

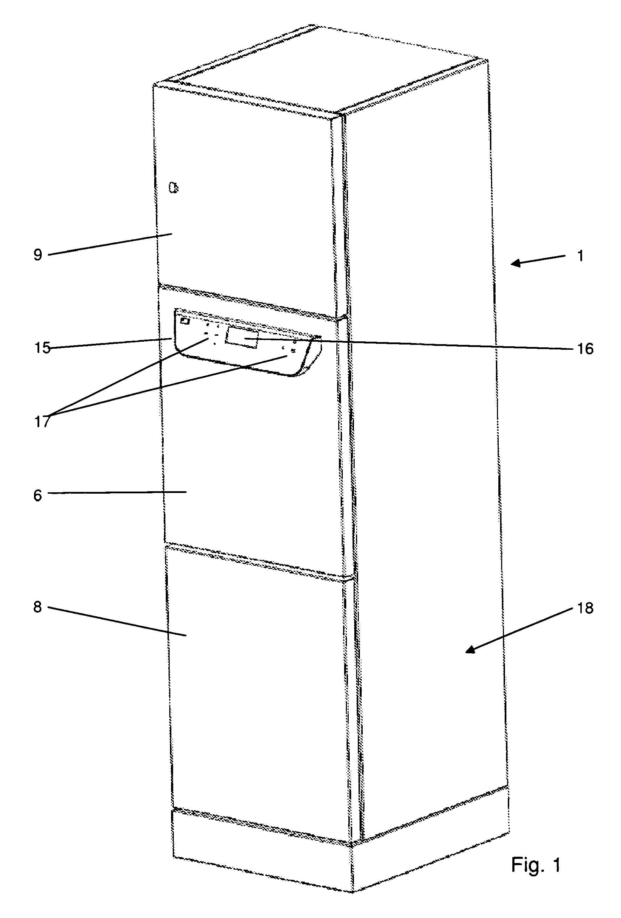

Die Figuren 1 bis 4 zeigen in jeweils unterschiedlicher Ansicht eine erfindungsgemäße Vorrichtung 1 zur Reinigung und thermischen Desinfektion von Spülgut 2. Als Spülgut 2 finden insbesondere Steckbecken 3 und Urinflaschen 4 Verwendung, weshalb die erfindungsgemäße Vorrichtung 1 auch als Steckbeckenspüler bezeichnet wird.

Die Vorrichtung 1 stellt eine Spülgutkammer 5 bereit. Im bestimmungsgemäßen Verwendungsfall nimmt die Spülgutkammer 5 das zu spülende Spülgut 2 auf.

Die Spülgutkammer 5 ist über eine Beschickungsöffnung zugänglich, die mittels einer Tür 6 verschließbar ausgebildet ist.

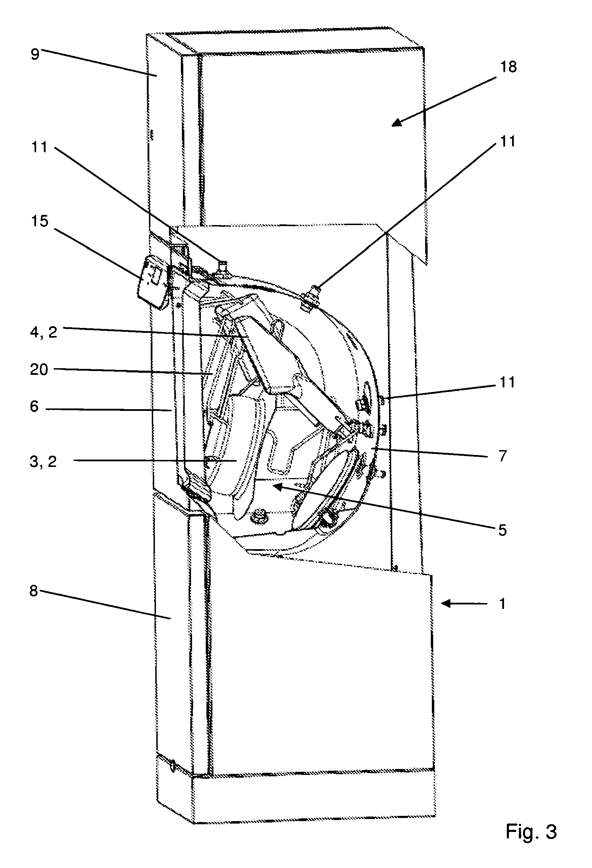

Wie insbesondere die teilgeschnittene Seitenansicht nach Fig. 3 erkennen lässt, ist die Spülgutkammer 5 durch einen vom Einsatz 7 bereitgestellten Hohlraum gebildet. Im bestimmungsgemäßen Verwendungsfall nimmt dieser Einsatz 7 das zu spülende Spülgut 2 auf.

Der Einsatz 7 stellt Düsen 11 bereit. Diese Düsen 11 sind -wie dies in den Zeichnungen der Übersicht wegen nicht weiter dargestellt ist - an Wasserzuführungsleitungen angeschlossen. Über diese gelangt im bestimmungsgemäßen Verwendungsfall Wasser an die Düsen 11, mittels welcher ein Aufsprühen von Wasser auf das zu reinigende Spülgut 2 erfolgen kann.

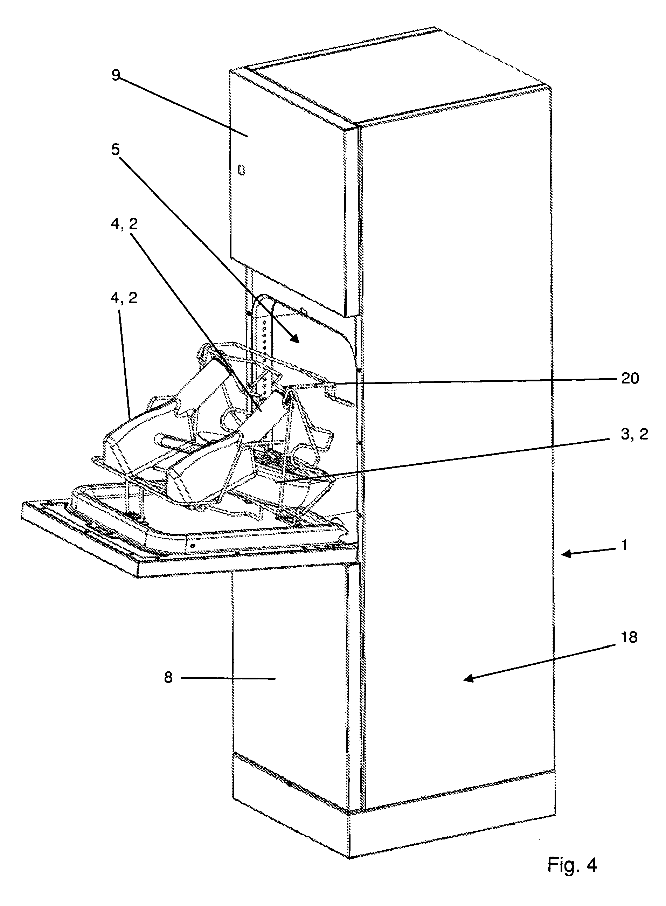

Innenseitig der die Spülgutkammer 5 verschließenden Tür 6 ist ein Drahtgestell 20 angeordnet. Im bestimmungsgemäßen Verwendungsfall nimmt dieses Drahtgestell 20 das zu reinigende Spülgut 2 auf. Wie insbesondere eine Zusammenschau der Figuren 3 und 4 erkennen lässt, ist das Drahtgestell 20 bei geöffneter Tür 6 zugänglich, d. h., es kann mit zu reinigendem Spülgut 2 bestückt werden bzw. gereinigtes Spülgut 2 kann der Vorrichtung 1 entnommen werden.

Zwecks Betätigung der Tür 6 verfügt diese über eine Bedieneinrichtung 15. Diese ist im gezeigten Ausführungsbeispiel nach Art eines Griffes ausgebildet. Mit Bezug auf die Zeichnungsebene nach Fig. 1 weist die Bedieneinrichtung 15 oberseitig ein Display 16 und mehrere Bedienelemente 17 auf. Über die Bedienelemente 17 kann eine Auswahl des von der erfindungsgemäßen Vorrichtung 1 durchzuführenden Reinigungsprogramms durchgeführt werden. Das Display 16 ist bevorzugterweise als Anzeigeeinrichtung ausgestaltet und bietet die Möglichkeit, beispielsweise den Fortschritt einer Verfahrensdurchführung anzuzeigen.

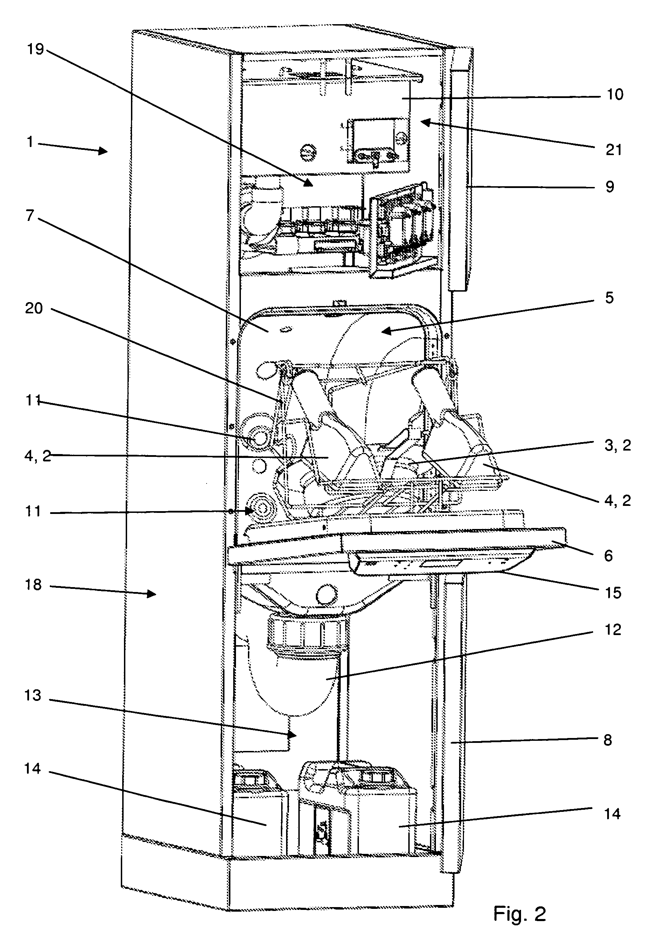

Der die Spülgutkammer 5 bereitstellende Einsatz 7 ist von einem Gehäuse 18 aufgenommen. Dieses Gehäuse 18 stellt ferner einen unterhalb des Einsatzes 7 ausgebildeten Stauraum 13 zur Verfügung, wie insbesondere die Darstellung nach Fig. 2 erkennen lässt. Der Stauraum 13 kann beispielsweise zur Aufnahme von Kanistern 14 dienen.

Der Stauraum 13 dient ferner der Unterbringung eines Siphons 12 einer an den Einsatz 7 angeschlossenen und in den Figuren nicht näher dargestellten Wasserabführungsleitung. Der Stauraum 13 ist über eine Tür 8 zugänglich.

Mit Bezug auf die Zeichnungsebene nach Fig. 2 ist oberhalb des die Spülgutkammer 5 bereitstellenden Einsatzes 7 ein weiterer Aufnahmeraum 21 vorgesehen, der mittels einer Tür 9 verschließbar ausgebildet ist. Der Aufnahmeraum 21 nimmt einen Dampferzeuger 10 sowie eine Steuereinrichtung 19 auf.

Die Steuereinrichtung 19 dient dem automatischen Öffnen und Schließen der Tür 6, wobei die Steuereinrichtung 19 erfindungsgemäß derart ausgebildet ist, dass nach Abschluss einer Beaufschlagung des Spülguts 2 mit Dampf aus dem Dampferzeuger 10 ein automatisches Öffnen der Tür 6 um ein vorgebbares Spaltmaß erfolgt.

Die erfindungsgemäße Verfahrensdurchführung geht wie folgt vonstatten:
Das von der Tür 6 getragene Drahtgestell 20 wird mit zu reinigenden Steckbecken 3 und/oder Urinflaschen 4 bestückt. Alsdann wird die Tür 4 verschlossen, infolgedessen die Steckbecken 3 und/oder Urinflaschen 4 in die vom Einsatz 7 bereitgestellte Spülgutkammer 5 einschwenken, wie dies die Darstellung nach Fig. 3 zeigt.

Es erfolgt sodann der Verfahrensschritt der Reinigung. Die Reinigung erfolgt vorzugsweise mittels Kaltwasser. Aber auch der Einsatz von zuvor aufgewärmtem Wasser ist natürlich möglich. In jedem Fall wird das Wasser über die Düsen 11 auf das zu reinigende Spülgut 2 aufgebracht. Dies kann unter Druck erfolgen. Infolge des Wasserauftrags wird das Spülgut 2 gereinigt, d. h. von Anhaftungen und/oder dergleichen befreit.

Das über die Düsen 11 ausgebrachte Wasser wird zusammen mit den vom Spülgut 2 entfernten Anhaftungen über den Siphon 12 in die Abwasserleitung gefördert und abtransportiert.

Nach erfolgtem Reinigungsschritt wird in einem zweiten Verfahrensschritt eine thermische Desinfektion des gereinigten Spülgutes 2 vorgenommen. Hierzu dient der Dampferzeuger 10.

Der mittels des Dampferzeugers 10 erzeugte Dampf wird in die Spülgutkammer 5 eingeleitet. Infolgedessen kommt es zu einer thermischen Desinfektion des Spülgutes 2.

Nach erfolgter Desinfektion des Spülgutes 2 erfolgt in einem dritten und letzten Verfahrensschritt eine Rückkühlung des Spülguts 2. Zu diesem Zweck wird erfindungsgemäß die die Spülgutkammer 5 verschließende Tür 6 um ein vorgebbares Spaltmaß automatisch geöffnet. Durch den so entstehenden Öffnungsspalt kann die Dampfatmosphäre aus der Spülgutkammer 5 in die Umgebungsatmosphäre entweichen. Hierdurch setzt ein Selbstkühlungsprozess des Spülgutes 2 ein.

Der Kühlprozess kann dadurch gesteigert werden, dass bei geöffneter Tür 6 Zuluft in die Spülgutkammer 5 eingebracht wird. Dies erzeugt einen zusätzlichen Kühleffekt und trägt im Übrigen zu einem schnelleren Austrag der Dampfatmosphäre bei.

Bevorzugterweise wird dem Dampferzeuger 10 ein aromatisiertes Entkalkungsmittel zugeführt. Bei der Dampferzeugung entsteht sodann ein aromatisierter Dampf. Dieser gelangt bei einem Austrag während der Trocknungsphase in die die Vorrichtung 1 umgebende Atmosphäre, womit diese geruchlich aufgewertet wird.

## Patentansprüche

1. Verfahren zur Reinigung und thermischen Desinfektion von Spülgut (2) wie Steckbecken (3), Urinflaschen (4) und dergleichen, bei dem das in einer mittels einer Tür (6) verschließbaren Spülgutkammer (5) befindliche Spülgut (2) in einem ersten Verfahrensschritt zwecks Reinigung mit einer Reinigungsflüssigkeit und in einem zweiten Verfahrensschritt zwecks thermischer Desinfektion mit Wasserdampf beaufschlagt wird, wobei nach Abschluss des Desinfektionsschrittes eine Rückkühlung des Spülguts (2) erfolgt, zu welchem Zweck die die Spülgutkammer (5) verschließende Tür (6) um ein vorgebbares Spaltmaß automatisch geöffnet wird,
**dadurch gekennzeichnet,**
**dass** die Tür (6) in ihrer Öffnungsstellung für eine vorgebbare Zeitspanne gehalten und anschließend für eine manuelle oder automatische Türöffnung freigegeben wird.

2. Verfahren nach Anspruch 1,
**dadurch gekennzeichnet,**
**dass** nach einem automatischen Öffnen der Tür (6) Zuluft in die Spülgutkammer (5) eingebracht wird.

3. Verfahren nach einem der vorhergehenden Ansprüche,
**dadurch gekennzeichnet,**
**dass** die Dampferzeugung zur thermischen Desinfektion unter Verwendung eines aromatisierten Wasserzusatzes erfolgt.

## Claims

1. Method for cleaning and thermally disinfecting washware (2), such as bedpans (3), urine bottles (4) and the like, in which method the washware (2), located in a ware chamber (5) which is closed by a door (6), is exposed to a cleaning liquid in a first method step for the purpose of cleaning, and is exposed to steam in a second method step for the purpose of thermal disinfection, the washware (2) being cooled after the disinfection step is completed, the door (6) which closes the ware chamber (5) being automatically opened by a gap of a predetermined size for this purpose, **characterised in that** the door (6) is held in its open position for a predetermined period of time and is subsequently released for manual or automatic door opening.

2. Method according to claim 1, **characterised in that** fresh air is introduced into the ware chamber (5) after automatic opening of the door (6).

3. Method according to any of the preceding claims, **characterised in that** the steam for the thermal disinfection is generated by adding scented water.

## Revendications

1. Procédé de nettoyage et de désinfection thermique d'éléments à laver (2) tels que des bassins de lit (3), des urinaux (4) et similaires, dans lequel les éléments à laver (2) situés dans une chambre d'éléments à laver (5) pouvant être fermée au moyen d'une porte (6) reçoivent dans une première étape de procédé, à des fins de nettoyage, un liquide de nettoyage et, dans une deuxième étape de procédé, à des fins de désinfection thermique, reçoivent de la vapeur d'eau, un refroidissement en retour des éléments à laver (2) étant effectué après l'achèvement de l'étape de désinfection et, dans ce but, la porte (6) fermant la chambre d'éléments à laver (5) étant ouverte automatiquement sur une distance d'interstice paramétrable,
**caractérisé en ce que**
la porte (6) est maintenue dans sa position d'ouverture pendant une période paramétrable et est libérée ensuite pour une ouverture de porte manuelle ou automatique.

2. Procédé selon la revendication 1,
**caractérisé en ce que**,
après une ouverture automatique de la porte (6), de l'air frais est introduit dans la chambre d'éléments à laver (5).

3. Procédé selon une des revendications précédentes,
**caractérisé en ce que**
la production de vapeur pour la désinfection thermique est effectuée en utilisant un apport d'eau aromatisé.
